# FASCICULE DE BREVET EUROPEEN

(11) **EP 0 941 046 B1**
(45) Date de publication et mention de la délivrance du brevet: **22.05.2002**
(21) Numéro de dépôt: 97913246.1
(22) Date de dépôt: 04.11.1997
(51) Int. Cl.: A61K 7/09

(54) **COMPOSITION POUR LA DEFORMATION PERMANENTE DES FIBRES KERATINIQUES, OBTENTION ET MISE EN OEUVRE**
ZUSAMMENSETZUNG ZUR DAUERHAFTEN VERFORMUNG KERATINISCHER FASERN, IHRE HERSTELLUNG UND ANWENDUNG
COMPOSITION FOR THE PERMANENT DEFORMATION OF KERATINOUS FIBERS, METHOD OF PRODUCTION AND APPLICATION

(30) Priorité: 06.11.1996 FR 9613544
(43) Date de publication de la demande: 15.09.1999
(73) Titulaire: EUGENE PERMA, 75009 Paris (FR)
(72) Inventeur: GALERNE, Jean, F-93160 Noisy-le-Grand (FR); SAINT-MARTIN, Monique, F-75012 Paris (FR); MAZOYER, Monique, F-77410 Villevaude (FR)
(74) Mandataire: Phélip, Bruno
(86) Numéro de dépôt international: FR9701974
(87) Numéro de publication internationale: WO9819657

(56) Documents cités:
- EP-A- 0 114 414
- WO-A-92/17155
- GB-A- 941 300
- DATABASE WPI Week 8133 Derwent Publications Ltd., London, GB; AN 81-59656D XP002036481 "Permanent wave soln. for hair treatment - contains thioglycolic acid salt, N-acylaminoacid salt to inhibit damage to hair" & JP 56 079 618 A (AMOROS & TANABE SEIYAKU) , 30 juin 1981

## Description

La présente invention a pour objet une composition pour la déformation permanente des fibres kératiniques, et en particulier des cheveux.

Elle est en outre relative à un procédé d'obtention d'une telle composition.

Elle concerne également un procédé de mise en oeuvre d'une telle composition pour la déformation permanente des fibres kératiniques.

La déformation des cheveux s'effectue classiquement en deux étapes. Dans un premier temps (phase de réduction), les liaisons de cohésion de la fibre capillaire, principalement les ponts disulfures, sont rompues à l'aide d'une solution contenant un agent réducteur. Après rinçage de la chevelure, les cheveux sont fixés avec la déformation souhaitée à l'aide d'une solution oxydante qui reconstitue les liaisons cystéiques et confère aux cheveux une déformation durable (phase de fixation ou d'oxydation). Ce procédé permet de friser des cheveux naturellement raides (ondulation permanente utilisant des bigoudis) ou de défriser des cheveux bouclés à l'origine (défrisage par lissage).

Les produits de défrisage peuvent être formulés comme des produits d'ondulation permanente, seule la nouvelle forme imposée aux cheveux varie. Dans le cas d'une ondulation permanente (couramment appelée permanente) on utilise des bigoudis pour imposer une forme bouclée aux cheveux. Dans le cas d'un défrisage, on « raidit » les cheveux en les lissant avec un peigne.

Pour faciliter l'application du produit et l'étirement des cheveux à plat, les compositions défrisantes sont formulées dans un support épais (gel ou crème) à la différence des liquides d'ondulation qui se présentent le plus souvent sous la forme liquide.

L'agent réducteur le plus généralement utilisé est le sel d'ammonium de l'acide thioglycolique. La concentration maximale en acide thioglycolique est légalement limitée à 11 % dans de nombreux pays. Une efficacité de frisure maximale peut être assurée par l'addition de sel d'ammonium de l'acide thiolactique.

Comme la majorité des mercaptans, ces deux agents réducteurs présentent un potentiel d'ionisation relativement important. Ils ne peuvent donc avoir une action suffisante qu'à pH élevé (voisin de 9 pour le thioglycolate d'ammonium). L'efficacité des solutions réductrices est ainsi fonction de la combinaison pH-concentration d'acide thioglycolique, la baisse de l'un des facteurs devant être impérativement compensée par un accroissement du deuxième. Pour une activité égale, un abaissement du pH nécessite donc obligatoirement une hausse de la concentration en acide thioglycolique.

Ces deux facteurs présentent de nombreux inconvénients tant pour les cheveux que pour l'utilisateur.

Le pH fortement basique altère les propriétés de surface du cheveu. Il en résulte une augmentation du coefficient de friction qui affecte le démêlage et la douceur, et un déséquilibre ionique qui se traduit par une augmentation du caractère anionique du cheveu.

Au fil des applications, la forte teneur en agent réducteur affaiblit considérablement la fibre capillaire, ce qui induit une augmentation du nombre de liaisons non reconstituées et une perte en soufre.

Après des traitements répétés, le cheveu ne retrouve pas son intégrité d'origine et perd peu à peu ses propriétés mécaniques: souplesse, élasticité, résistance à la traction. Ces dégradations physico-chimiques sont particulièrement sensibles au toucher des cheveux qui deviennent rêches et difficilement démélables au coiffage.

Le pH est généralement ajusté à l'aide d'ammoniaque ou d'une autre base forte comme la monoéthanolamine par exemple. L'ammoniaque a une forte odeur caractéristique et a la propriété d'exhaler celle également peu agréable de l'acide thioglycolique d'autant plus que sa concentration est élevée. Il en résulte un inconfort d'utilisation touchant les personnes traitées et les personnes effectuant le traitement.

L'acide thioglycolique relativement bien toléré à faible dose, voit son pouvoir irritant augmenter de façon proportionnelle à sa concentration, cette intolérance cutanée étant exacerbée par l'effet du pH.

Des solutions ont été proposées pour pallier ces inconvénients. Ainsi, la demande FR 70 20 797 propose de remplacer l'excès d'ammoniaque par un carbonate d'un sel sodique d'un aminoacide, dans des solutions pour l'ondulation permanente à base d'acide thioglycolique ou de thioglycolate d'ammonium. Un tel aminoacide peut entre autres être la méthionine.

Dans d'autres brevets, de l'urée a été utilisée à titre d'agent réducteur. Ainsi, le brevet FR 1 293 385 décrit une composition contenant du chlorhydrate de cystéine, de l'uréase et de l'urée. L'uréase va dégrader l'urée, conduisant ainsi à la formation d'ammoniaque. De même, dans le brevet US 3 242 052 de la N-acétyl-L-cystéine est utilisée en présence d'urée et d'un copolymère de polyoxyéthylène-polyoxypropylène.

Néanmoins, ces solutions doivent être améliorées en particulier car elles nécessitent des temps d'action très longs et sont mises en oeuvre à des pH élevés.

La demanderesse s'est donc attachée à rechercher une composition permettant d'obtenir une ondulation permanente de manière rapide et reproductible, et ne lésant pas les fibres kératiniques.

Elle a montré que la combinaison de l'altantoïne, de la cystéine et de la méthionine permet d'optimiser l'activité des agents réducteurs classiquement utilisés et de ce fait de diminuer l'agression subie par les fibres capillaires lors des traitements de déformation permanente.

La présente invention est donc relative à une composition pour la déformation permanente des fibres kératiniques, que ce soit pour les onduler ou les défriser, et en particulier des cheveux, présentant un pH proche de la neutralité, et comprenant au moins un acide mercaptocarboxylique, ou l'un de ses sels ou dérivés, une base forte, telle que de l'ammoniaque, et une combinaison en quantités efficaces d'allantoïne, de cystéine et de méthionine, ou de leurs dérivés, sels ou esters.

Pour la présente invention on entend par dérivé d'une molécule donnée toute autre molécule ayant une structure proche et une fonction similaire, sans pour autant qu'elle en soit obligatoirement un métabolite ou un catabolite.

Ainsi, dans la composition selon la présente invention, l'allantoïne peut être substituée par toute molécule ayant un caractère fortement hygroscopique permettant une hydratation rapide et maximale du cheveu, qui facilite la rupture des liaisons hydrogène et l'accessibilité des ponts disulfure aux agents réducteurs.

Des dérivés de la méthionine peuvent être ses sels, ses esters ou ses dérivés acétylés comme par exemple le chlorhydrate de méthionine ou la N-acétyl méthionine.

Des dérivés de la cystéine peuvent être ses sels, ses esters ou ses dérivés acétylés comme par exemple le chlorhydrate de cystéine ou la N-acétyl cystéine.

De manière préférentielle, ladite composition est sous la forme de deux compositions, liquide ou solide, qui sont mélangées extemporanément.

Les acides mercaptocarboxyliques sont choisis parmi ceux pouvant être appliqués sur les fibres kératiniques, sans dommage pour ces dernières.

De manière avantageuse, ladite composition comprend, à titre d'acide mercaptocarboxylique, de l'acide thioglycolique et éventuellement de l'acide thiolactique. Les acides peuvent être sous la forme d'un de leurs sels, en particulier leurs sels d'ammonium. De manière préférentielle, ces acides seront présents dans la composition à raison d'environ 2 à 20% en poids.

Une telle composition peut comprendre entre 2 et 15%, préférentiellement entre 5 et 11% en poids d'un sel d'acide thioglycolique, tel que son sel d'ammonium. Elle peut aussi comprendre jusqu'à 5%, préférentiellement entre 1 et 3% en poids d'un sel d'acide thiolactique, tel que son sel d'ammonium.

Avantageusement, la quantité d'allantoïne est comprise entre 0,1 et 1%, préférentiellement entre 0,3 et 0,6% en poids de la composition, tandis que la cystéine est comprise à raison de 0,2 à 3%, préférentiellement 0,4 à 1% en poids de la composition.

La méthionine est quant à elle préférentiellement présente à raison de 0,1 à 2%, préférentiellement 0,3 à 1% en poids de la composition.

De manière avantageuse, une telle composition présente un pH compris entre 7 et 8, préférentiellement entre 7,2 et 7,8 et encore plus préférentiellement compris entre 7,4 et 7,5.

La présente composition peut comprendre de 0,05 à 1% en poids d'ammoniaque.

Elle peut aussi contenir des séquestrants, des conditionneurs, des parfums et des opacifiants.

De manière particulièrement avantageuse, une composition selon la présente invention comprend:
- environ 11% en poids de thioglycolate d'ammonium,
- environ 2% en poids de thiolactate d'ammonium,
- environ 0,1% en poids d'ammoniaque,
- environ 5% en poids de bicarbonate d'ammonium,
- environ 1% en poids de cystéine,
- environ 0,4 % en poids d'allantoïne, et
- environ 0,5% en poids de méthionine.

La composition selon la présente invention permet d'utiliser des concentrations en acides mercaptocarboxyliques plus faibles que celles utilisées dans des compositions présentant le même pH et ne contenant pas la combinaison de l'allantoïne, de la méthionine et de la cystéine. En effet, la combinaison de ces trois composés augmente l'efficacité des réducteurs et permet de réduire la concentration de thioglycolate de 1 à 4%, de supprimer le thiolactate, et ainsi de limiter les inconvénients inhérents aux compositions de l'état de la technique.

La possibilité de diminuer les taux de mercaptans limite les dégradations physico-chimiques du cheveux et l'inconfort causé par l'odeur très désagréable de ces composés. En particulier, il a été constaté, après des tests effectués sur des modèles, qu'il n'y avait pas réapparition de mauvaises odeurs lors des shampooings suivant le traitement, contrairement aux déformations permanentes obtenues avec des compositions antérieurement connues.

Les compositions selon la présente invention sont de plus particulièrement adaptées pour le traitement de cheveux sensibilisés. On définit classiquement ainsi des cheveux fragilisés par des traitements physiques ou chimiques antérieurs, qui ont modifié les propriétés de résistance caractéristiques de cheveux naturels vierges. Ces traitements peuvent être des permanentes, défrisages, colorations, décolorations, brossages ou chauffages excessifs qui, en usage répété, altèrent les propriétés physico-chimiques du cheveu.

Une composition selon la présente invention est obtenue par mélange des divers composés la constituant.

Néanmoins, du fait de leurs instabilités, l'allantoïne et la méthionine sont avantageusement ajoutées extemporanément à une solution contenant les autres ingrédients de la composition.

La présente invention est donc relative à un procédé de fabrication d'une telle composition caractérisé en ce que l'on ajoute extemporanément l'allantoïne et la méthionine, ou leurs dérivés, sous forme de poudres, à une composition aqueuse présentant un pH substantiellement neutre et contenant au moins un acide mercaptocarboxylique, une base forte, telle que de l'ammoniaque, et de la cystéine. Une telle composition aqueuse constitue un autre objet de la présente invention.

La présente invention a en outre pour objet un procédé de déformation permanente des fibres kératiniques, et en particulier des cheveux, comprenant les étapes suivantes:
- application sur les fibres préalablement mises en forme d'une composition selon la présente invention pendant une durée permettant la réduction des liaisons assurant la cohésion des fibres,
- rinçage des fibres,
- application d'une solution oxydante pendant une durée permettant la reconstitution des liaisons assurant la cohésion des fibres, et
- rinçage et séchage des fibres.

De manière avantageuse, la solution oxydante contient du peroxyde d'hydrogène, à raison de 0,5 à 13%, préférentiellement 1 à 6% et encore plus préférentiellement 2% en poids environ. Son pH est de préférence compris entre 3 et 4. Une autre composition oxydante peut contenir un bromate alcalin et dans ce cas son pH sera préférentiellement proche de la neutralité.

De manière avantageuse, la composition selon la présente invention, ou composition réductrice, est appliquée pendant 5 à 30 minutes, et préférentiellement pendant 10 à 20 minutes selon la nature des cheveux et la température ambiante.

La composition oxydante peut quant à elle être appliquée pendant environ 1 à 10 minutes, et préférentiellement pendant 3 à 7 minutes environ.

Les cheveux ainsi traités présentent une frisure homogène, les boucles formées étant identiques sur toute la longueur du cheveu. Ce procédé leur confère de la brillance et un toucher soyeux. Ils restent hydratés et se démêlent facilement.

La présente invention est illustrée sans pour autant être limitée par les exemples qui suivent :

### EXEMPLE 1:

### Obtention d'une ondulation permanente sur cheveux naturels par application d'une composition selon l'invention.

Sur cheveux mouillés, préalablement enroulés sur des bigoudis, on applique la composition réductrice obtenue en ajoutant 1g de composition B à 75g de composition A.

| Composition (A) | |
|---|---|
| Thioglycolate d'ammonium à 50% | 22,00 g |
| Thiolactate d'ammonium à 50% | 4,00 g |
| Ammoniaque 20% | 0,60 g |
| Bicarbonate d'ammonium | 5,00 g |
| Cystéine | 1,00 g |
| Ammoniaque 20 % | qsp pH = 7,4 |
| Eau déminéralisée | qsp 100g |

| Composition (B): | |
|---|---|
| Allantoïne | 30,00 g |
| Méthionine | 40,00 g |
| Excipient: poudre de silice | qsp 100 g |

On laisse agir pendant 18 min, puis on rince à l'eau avant d'appliquer la composition oxydante C.

| Composition (C): | |
|---|---|
| Peroxyde d'hydrogène en solution 50% | 4,33g |
| Acide citrique | qsp pH 3,5 |
| Eau déminéralisée | qsp 100 g |

Après avoir laissé agir la composition oxydante pendant 5 min, on rince à l'eau, on enlève les bigoudis et sèche les cheveux.

On ne constate pas d'apparition de mauvaises odeurs dans le temps, même sur cheveux remouillés.

### EXEMPLE 2

### Obtention d'une ondulation permanente sur cheveux sensibilisés par application d'une composition selon l'invention.

Des cheveux sensibilisés sont traités d'une manière similaire à l'exemple 1. Seule la composition A varie.

| **Composition (A)** | |
|---|---|
| Thioglycolate d'ammonium à 50% | 13,00 g |
| Ammoniaque 20% | 0,60 g |
| Bicarbonate d'ammonium | 4,00 g |
| Cystéine | 1,00 g |
| Ammoniaque 20% | qsp pH = 7.4 |
| Eau déminéralisée | qsp 100 g |

### EXEMPLE 3:

### Comparaison d'une composition selon la présente invention et des compositions de l'état de la technique.

La composition décrite dans l'exemple 1 a été comparée avec diverses compositions d'ondulations permanentes de l'état de la technique.

Il ressort de ces comparaisons que l'utilisation de compositions ne contenant pas d'allantoïne ni de méthionine fournit une frisure plus relâchée et moins tonique qu'en présence de ces deux composés, avec une tenue de boucle inférieure dans le temps. La différence est particulièrement flagrante sur des cheveux séchés.

L'utilisation d'une composition dépourvue de méthionine, mais contenant de la cystéine et de l'allantoïne a conduit à une frisure moins nerveuse que celle obtenue avec la composition selon l'exemple 1.

L'apport de méthionine influe favorablement non seulement sur la nervosité de la frisure mais également sur le démêlage des cheveux permanentés qui est facilité.

L'utilisation d'une composition dépourvue d'allantoïne donne des résultats moins satisfaisants en qualités de nervosité et tenue de boucle (« curl rétention »).

Grâce à l'association des trois composés, la diminution des teneurs en mercaptans permet d'obtenir une frisure nerveuse et durable tout en préservant les qualités de douceur et de démêlage des cheveux. Elle offre aussi l'avantage d'améliorer le confort d'utilisation en diminuant les risques d'irritation et l'odeur très désagréable des composés réducteurs.

## Revendications

1. Composition pour la déformation permanente des fibres kératiniques, et en parliculier des cheveux, présentant un pH compris entre 7 et 8 et comprenant au moins un acide mercaptocarboxylique, ou l'un de ses sels ou dérivés, une base forte et une combinaison en quantités efficaces d'allantoïne, de cystéine, et de méthionine, ou de leurs dérivés, sels ou esters.

2. Composition selon la revendication 1 **caractérisée en ce qu'**elle est sous la forme de deux compositions, liquides ou solides, qui sont mélangées extemporanément.

3. Composition selon l'une des revendications 1 et 2 **caractérisée en ce qu'**elle comprend à titre d'acide mercaptocarboxylique, de l'acide thioglycolique, de l'acide thiolactique, ou leurs sels d'ammonium.

4. Composition selon l'une des revendications 1 à 3 **caractérisée en ce qu'**elle comprend entre 2 et 20% en poids d'acide mercaptocarboxylique.

5. Composition selon l'une des revendications 1 à 4 **caractérisée en ce qu'**elle comprend entre 2 et 15%, préférentiellement entre 5 et 11% en poids d'un sel de l'acide thioglycolique, tel que son sel d'ammonium.

6. Composition selon l'une des revendications 1 à 5 **caractérisée en ce qu'**elle comprend jusqu'à 5%, préférentiellement entre 1 et 3 % en poids d'un sel dé l'acide thiolactique, tel que son sel d'ammonium.

7. Composition selon l'une des revendications 1 à 6 **caractérisée en ce qu'**elle comprend entre 0,1 et 1 %, préférentiellement entre 0,3 et 0,6% en poids d'allantoïne.

8. Composition selon l'une des revendications 1 à 7 **caractérisée en ce qu'**elle comprend entre 0,2 et 3%, préférentiellement entre 0,4 et 1% en poids de cystéine.

9. Composition selon l'une des revendications 1 à 8 **caractérisée en ce qu'**elle comprend entre 0,1 et 2%, préférentiellement entre 0,3 et 1% en poids de méthionine.

10. Composition selon l'une des revendications 1 à 9 **caractérisée en ce que** la base forte est l'ammoniaque.

11. Composition selon l'une des revendications 1 à 10 **caractérisée en ce qu'**elle présente un pH compris entre 7,2 et 7,8 et préférentiellement entre 7,4 et 7,5.

12. Composition selon l'une des revendications 1 à 11 **caractérisée en ce qu'**elle comprend:
- environ 11% en poids de thioglycolate d'ammonium,
- environ 2% en poids de thiolactate d'ammonium,
- environ 0,1 % en poids d'ammoniaque,
- environ 5% en poids de bicarbonate d'ammonium,
- environ 1% en poids de cystéine,
- environ 0,4% en poids d'allantoïne, et
- environ 0,5% en poids de méthionine.

13. Procédé de fabrication d'une composition selon l'une des revendications 1 à 12 **caractérisé en ce que** l'on ajoute extemporanément l'allantoïne et la méthionine, ou leurs dérivés, sous forme de poudres à une solution aqueuse présentant un pH compris entre 7 et 8, contenant au moins un acide mercaptocarboxylique, ou l'un de ses sels ou l'un de ses dérivés, une base forte et de la cystéine, ou l'un de ses dérivés, sels ou esters.

14. Composition pour la mise en oeuvre du procédé selon la revendication 13 présentant un pH compris entre 7 et 8 et comprenant au moins un acide mercaptocarboxylique ou l'un de ses sels ou dérivés, une base forte et de la cystéine, ou l'un de ses dérivés, sels ou esters.

15. Procédé pour la déformation permanente des fibres kératiniques, et en particulier des cheveux, comprenant les étapes suivantes:
- application sur les fibres, préalablement mises en forme, d'une composition selon l'une des revendications 1 à 12, pendant une durée permettant la réduction des liaisons assurant la cohésion des fibres,
- rinçage des fibres,
- application d'une solution oxydante pendant une durée permettant la reconstitution des liaisons assurant la cohésion des fibres, et
- rinçage et séchage des fibres.

16. Procédé selon la revendication 15 **caractérisé en ce que** la solution oxydante contient du peroxyde d'hydrogène, ou un bromate alcalin.

17. Procédé selon l'une des revendications 15 et 16 **caractérisé en ce que** la composition selon l'une des revendications 1 à 12 est appliquée pendant 5 à 30 minutes, préférentiellement pendant 10 à 20 minutes en fonction de la nature des cheveux et de la température ambiante.

18. Procédé selon l'une des revendications 15 et 17 **caractérisé en ce que** la composition oxydante est appliquée pendant 1 à 10 minutes, préférentiellement pendant 3 à 7 minutes.

19. Procédé selon l'une des revendications 15 à 18 **caractérisé en ce que** la composition selon l'une des revendications 1 à 12 est préparée extemporanément, avant application, par le procédé selon la revendication 13.

## Claims

1. Composition for the permanent deformation of keratin fibres, and in particular hair, with a pH of between 7 and 8 and comprising at least a mercaptocarboxylic acid, or one of its salts or derivatives, a strong base and a combination in effective quantities of allantoin, cysteine, and methionine, or of their derivatives, salts or esters.

2. Composition according to claim1, **characterized in that** it is in the form of two liquid or solid compositions which are mixed extemporaneously.

3. Composition according to one of claims 1 and 2, **characterized in that** it contains, as mercaptocarboxylic acid, thioglycolic acid, thiolactic acid, or their ammonium salts.

4. Composition according to one of claims 1 to 3, **characterized in that** it contains between 2 and 20% by weight of mercaptocarboxylic acid.

5. Composition according to one of claims 1 to 4, **characterized in that** it contains between 2 and 15%, preferably between 5 and 11% by weight of a salt of thioglycolic acid, such as its ammonium salt.

6. Composition according to one of claims 1 to 5, **characterized in that** it contains up to 5%, preferably between 1 and 3% by weight of a salt of thiolactic acid, such as its ammonium salt.

7. Composition according to one of claims 1 to 6, **characterized in that** it contains between 0.1 and 1%, preferably between 0.3 and 0.6% by weight of allantoin.

8. Composition according to one of claims 1 to 7, **characterized in that** it contains between 0.2 and 3%, preferably between 0.4 and 1% by weight of cysteine.

9. Composition according to one of claims 1 to 8, **characterized in that** it contains between 0.1 and 2%, preferably between 0.3 and 1% by weight of methionine.

10. Composition according to one of claims 1 to 9, **characterized in that** the strong base is ammonia.

11. Composition according to one of claims 1 to 10, **characterized in that** it has a pH of between 7.2 and 7.8 and preferably between 7.4 and 7.5.

12. Composition according to one of claims 1 to 11, **characterized in that** it contains :
- about 11% by weight of ammonium thioglycolate,
- about 2% by weight of ammonium thiolactate,
- about 0.1% by weight of ammonia,
- about 5% by weight of ammonium bicarbonate,
- about 1 % by weight of cysteine,
- about 0.4% by weight of allantoin, and
- about 0.5% by weight of methionine.

13. Method of producing a composition according to one of claims 1 to 12, **characterized in that** allantoin and methionine, or their derivatives, are extemporaneously added in the form of powders to an aqueous solution with a pH of between 7 and 8 containing at least one mercaptocarboxylic acid, or one of its salts or derivatives, a strong base, and cysteine, or one of its derivatives, salts or esters.

14. Composition for use in the method according to claim 13, with a pH of between 7 and 8 and containing at least a mercaptocarboxylic acid, or one of its salts or derivatives, a strong base, and cysteine, or one of its derivatives, salts or esters.

15. Method for permanently deforming keratin fibres, and in particular hair, comprising the following steps :
- application to the fibres, previously placed in the desired shape, of a composition according to one of claims 1 to 12, for a duration sufficient to reduce the links ensuring the cohesion of the fibres,
- rinsing the fibres,
- application of an oxidizing solution for a duration sufficient to restore the links ensuring the cohesion of the fibres, and
- rinsing and drying the fibres.

16. Method according to claim 15, **characterized in that** the oxidizing solution contains hydrogen peroxide, or an alkaline bromate.

17. Method according to one of claims 15 and 16, **characterized in that** the composition according to one of claims 1 to 12 is applied for 5 to 30 minutes, preferably for 10 to 20 minutes as a function of the nature of the hair and the ambient temperature.

18. Method according to one of claims 15 to 17, **characterized in that** the oxidizing composition is applied for 1 to 10 minutes, preferably for 3 to 7 minutes.

19. Method according to one of claims 15 to 18, **characterized in that** the composition according to one of claims 1 to 12 is prepared extemporaneously, before application, by the method according to claim 13.

## Patentansprüche

1. Zusammensetzung für die permanente Verformung von Keratinfasern und insbesondere von Haaren, die einen pH-Wert zwischen 7 und 8 aufweist und wenigstens eine Mercaptocarboxylsäure oder eines ihrer Salze oder Derivate, eine starke Base und eine Kombination von wirksamen Mengen an Allantoin, Cystein und Methionin oder ihrer Derivate, Salze oder Ester umfasst.

2. Zusammensetzung gemäß Anspruch 1, **dadurch gekennzeichnet, dass** sie in Form von zwei flüssigen oder festen Zusammensetzungen vorliegt, die erst im Moment der Anwendung miteinander gemischt werden.

3. Zusammensetzung gemäß einem der Ansprüche 1 und 2, **dadurch gekennzeichnet, dass** sie als Mercaptocarboxylsäure Thioglycolsäure, Thiomilchsäure oder deren Ammoniumsalze umfasst.

4. Zusammensetzung gemäß einem der Ansprüche 1 bis 3, **dadurch gekennzeichnet, dass** sie zwischen 2 und 20 Gew.-% Mercaptocarboxylsäure umfasst.

5. Zusammensetzung gemäß einem der Ansprüche 1 bis 4, **dadurch gekennzeichnet, dass** sie zwischen 2 und 15 Gew.-%, vorzugsweise zwischen 5 und 11 Gew.-%, eines Salzes der Thioglycolsäure, wie ihres Ammoniumsalzes, umfasst.

6. Zusammensetzung gemäß einem der Ansprüche 1 bis 5, **dadurch gekennzeichnet, dass** sie bis zu 5 Gew.-%, vorzugsweise zwischen 1 und 3 Gew.-%, eines Salzes der Thiomilchsäure, wie ihres Ammoniumsalzes, umfasst.

7. Zusammensetzung gemäß einem der Ansprüche 1 bis 6, **dadurch gekennzeichnet, dass** sie zwischen 0,1 und 1 Gew.-%, vorzugsweise zwischen 0,3 und 0,6 Gew.-%, Allantoin umfasst.

8. Zusammensetzung gemäß einem der Ansprüche 1 bis 7, **dadurch gekennzeichnet, dass** sie zwischen 0,2 und 3 Gew.-%, vorzugsweise zwischen 0,4 und 1 Gew.-%, Cystein umfasst.

9. Zusammensetzung gemäß einem der Ansprüche 1 bis 8, **dadurch gekennzeichnet, dass** sie zwischen 0,1 und 2 Gew.-%, vorzugsweise zwischen 0,3 und 1 Gew.-%, Methionin umfasst.

10. Zusammensetzung gemäß einem der Ansprüche 1 bis 9, **dadurch gekennzeichnet, dass** es sich bei der starken Base um Ammoniak handelt.

11. Zusammensetzung gemäß einem der Ansprüche 1 bis 10, **dadurch gekennzeichnet, dass** sie einen pH-Wert zwischen 7,2 und 7,8 und vorzugsweise zwischen 7,4 und 7,5 aufweist.

12. Zusammensetzung gemäß einem der Ansprüche 1 bis 11, **dadurch gekennzeichnet, dass** sie folgendes umfasst:
- ungefähr 11 Gew.-% Ammoniumthioglycolat;
- ungefähr 2 Gew.-% Ammoniumthiolactat;
- ungefähr 0,1 Gew.-% Ammoniak;
- ungefähr 5 Gew.-% Ammoniumhydrogencarbonat;
- ungefähr 1 Gew.-% Cystein;
- ungefähr 0,4 Gew.-% Allantoin; und
- ungefähr 0,5 Gew.-% Methionin.

13. Verfahren zur Herstellung einer Zusammensetzung gemäß einem der Ansprüche 1 bis 12, **dadurch gekennzeichnet, dass** man Allantoin und Methionin oder ihre Derivate in Form von Pulvern erst im Moment der Anwendung in eine wässrige Lösung gibt, die einen pH-Wert zwischen 7 und 8 aufweist und wenigstens eine Mercaptocarboxylsäure oder eines ihrer Salze oder eines ihrer Derivate, eine starke Base sowie Cystein oder eines seiner Derivate, Salze oder Ester enthält.

14. Zusammensetzung zur Durchführung des Verfahrens gemäß Anspruch 13, die einen pH-Wert zwischen 7 und 8 aufweist und wenigstens eine Mercaptocarboxylsäure oder eines ihrer Salze oder eines ihrer Derivate, eine starke Base sowie Cystein oder eines seiner Derivate, Salze oder Ester enthält.

15. Verfahren zur permanenten Verformung von Keratinfasern und insbesondere von Haaren, das die folgenden Schritte umfasst:
- Auftragen einer Zusammensetzung gemäß einem der Ansprüche 1 bis 12 auf die Fasern, die zuvor in Form gebracht wurden, während einer Zeit, die die Reduktion der Bindungen ermöglicht, die den Zusammenhalt der Fasern gewährleisten;
- Abspülen der Fasern;
- Auftragen einer oxidierenden Lösung während einer Zeit, die die Rekonstitution der Bindungen ermöglicht, die den Zusammenhalt der Fasern gewährleisten; und
- Abspülen und Trocknen der Fasern.

16. Verfahren gemäß Anspruch 15, **dadurch gekennzeichnet, dass** die oxidierende Lösung Wasserstoffperoxid oder ein alkalisches Bromat enthält.

17. Verfahren gemäß einem der Ansprüche 15 und 16, **dadurch gekennzeichnet, dass** die Zusammensetzung gemäß einem der Ansprüche 1 bis 12 je nach der Art der Haare und der Umgebungstemperatur während 5 bis 30 Minuten, vorzugsweise während 10 bis 20 Minuten, aufgetragen wird.

18. Verfahren gemäß einem der Ansprüche 15 und 17, **dadurch gekennzeichnet, dass** die oxidierende Zusammensetzung während 1 bis 10 Minuten, vorzugsweise während 3 bis 7 Minuten, aufgetragen wird.

19. Verfahren gemäß einem der Ansprüche 15 bis 18, **dadurch gekennzeichnet, dass** die Zusammensetzung gemäß einem der Ansprüche 1 bis 12 erst im Moment der Anwendung, vor dem Auftragen, nach dem Verfahren gemäß Anspruch 13 hergestellt wird.
